# EUROPEAN PATENT APPLICATION

(11) **EP 4 372 677 A1**
(43) Date of publication of application: **22.05.2024**
(21) Application number: 22207542.6
(22) Date of filing: 15.11.2022
(51) Int. Cl.: G06T 7/00, G01N 23/04, G01N 33/02

(54) **SYSTEM AND METHOD FOR DETECTING AN UNWANTED STRUCTURE IN ONE OR MORE X-RAY IMAGES OF A FOOD PRODUCT**

(71) Applicant: InnospeXion ApS, 2690 Karlslunde (DK)
(72) Inventor: Basselbjerg, Kent, 2680 Solrød Strand (DK)
(74) Representative: AWA Denmark A/S

(57) **Abstract**

A method for detecting an unwanted structure in one or more X-ray images of a food product, the method comprising obtaining one or more X-ray images; processing the one or more X-ray images to find one or more areas of interest, each potentially comprising an unwanted structure; selecting for each of the one or more areas of interest a first group of pixels and a second group of pixels; processing the first group of pixels to determine a first parameter value and processing the second group of pixels to determine a second parameter value; and classifying the one or more X-ray images based on the first parameter value and the second parameter value, to determine the presence of an unwanted structure.

## Description

### Field

The present invention relates to a system and method for detecting an unwanted structure in one or more X-ray images of a food product.

### Background

Automatic handling and control of food products in modern food production is well established. The handling of food products may involve automatic cutting, sorting, and packaging of the food products. In any such automatic processes there is an inherent risk of error. External foreign bodies may be present or introduced into the food product, the food product may for example be a bad sample, a bone may be missed, or a bone splinter introduced by erroneously cutting. Any of the aforementioned scenarios involve unpleasant experiences for end consumers, but more importantly some of the scenarios are directly harmful or dangerous, as they may result in tooth injuries, cuts in the mouth or suffocation, especially if a child is the end consumer. To mitigate such risks X-ray detection systems have been implemented as a control measure in food production. X-ray offers good detection ability for high attenuation objects, such as bone.

EP3862748A1 discloses an X-ray inspection device for detecting residual bones in chicken breast meat, by use of a dual energy X-ray system combined with mapping of virtual regions of the chicken breast. However, even using such a device, certain unwanted structures or small objects may still be hard to detect and distinguish from their surroundings and normal structures of the food product. Thus, it remains a problem to provide an improved method of detecting unwanted structures and small objects in food products by use of X-ray detection.

### Summary

According to a first aspect the invention relates to a method for detecting an unwanted structure in one or more X-ray images of a food product, the method comprising; obtaining one or more X-ray images; processing the one or more X-ray images to find one or more areas of interest, each potentially comprising an unwanted structure; selecting for each of the one or more areas of interest a first group of pixels and a second group of pixels; processing the first group of pixels to determine a first parameter value and processing the second group of pixels to determine a second parameter value; and classifying the one or more X-ray images based on the first parameter value and the second parameter value, to determine the presence of an unwanted structure.

Consequently, a method that enables differentiating between detection of small structures, low attenuation structures and/or structures which resemble normally occurring structures, in food products is provided.

The food product may be any kind of food product appropriate for human or animal consumption. The food product may be a meat product, such as fish or poultry. The food product may e.g. be a meat fillet. The food product may be contained within a packaging, such as a paper wrapping, cardboard box, a metal can or glass jar.

The unwanted structure may be any foreign object or naturally occurring structure within a food product. The unwanted structure may be a bone or a part of a bone, including but not limited to compact bone tissue, cancellous bone tissue, subchondral bone tissue and cartilage. The unwanted structure may be tough tissue, such as tendons, ligaments, connective tissue, and scar tissue. The unwanted structure may be caused by wooden breast syndrome, e.g. in poultry meat. The unwanted structure may be an inorganic material, such as glass, metal, or stone.

The one or more X-ray images may be a single X-ray image obtained using a single X-ray energy level. The one or more X-ray images may be a plurality of X-ray images obtained using the same X-ray energy level for all of the plurality of X-ray images. The one or more X-ray images may be a plurality of X-ray images obtained using two or more X-ray energy levels. The one or more X-ray images may be obtained using a single X-ray tube and a single X-ray detector. The one or more X-ray images may be obtained using a single X-ray tube and two or more X-ray detectors. The one or more X-ray images may be obtained using two or more X-ray tubes and a single X-ray detector. The one or more X-ray images may be obtained using two or more X-ray tubes and two or more X-ray detectors. The one or more X-ray images may be obtained using one X-ray tube which outputs one energy level, and one or more attenuation filters which attenuates the energy level such that two or more energy levels may be detected.

The obtained one or more X-ray images may be loaded into a processing unit for immediate processing. Additionally or alternatively, the obtained one or more X-ray images may be loaded into a computer readable storage medium for later processing.

Typically, unwanted structures can be found in an X-ray image by looking for edges of the unwanted structures which will disturb X-rays travelling through the food product under investigation and give rise to high frequent spatial signals. Thus, processing of the one or more X-ray images to find one or more areas of interest may involve applying a filter to the one or more X-ray images which passes high frequent spatial signals i.e. spatial signals with a frequency above a predetermined threshold. Additionally or alternatively, the filter may be applied by first applying a filter to the one or more X-ray images which passes low frequent signals and then subsequently subtracting the resulting images from the original X-ray images. The filter may be a linear filter or non-linear filter. The processing may result in a binary image which can be used to find areas of interest. The binary image may be formed by using a threshold e.g. only filtered pixels having an intensity value above the threshold forms part of an area of interest.

The value of the threshold for a particular food product may be found experimentally. As an example, a training data set may be used containing a number of X-ray images of a particular food product such as 50, 100 or 200 images. Increasing the number of images will increase how precise a threshold may be determined. The training data set should preferably comprise a suitable mixture of X-ray images of the food product comprising an particular unwanted structure and X-ray images of the food product that do not contain the unwanted structure. It should preferably be determined for each X-ray image if the food product contains an unwanted structure and where in the food product the unwanted structure is present. This may be done by manually inspecting the X-ray images, using an alternative imaging modality, and / or by manually dissecting the food products.

The threshold should preferably be set so that all unwanted structures are identified as a potential area of interest. The threshold should preferably be set so that a reasonable number of false positives are allowed to secure that all unwanted structures are identified as a potential area of interest.

Areas of interest are portions of the one or more X-ray images which potentially comprises an unwanted structure. An area of interest may be defined by a group of pixels of the X-ray image representing the portion of the X-ray image which potentially comprises the unwanted structure.

The first group of pixels may comprise all the pixels from an area of interest. The first group of pixels may comprise a subgroup of the pixels from an area of interest, i.e. a lower number of pixels than the number of pixels in the area of interest.

The second group of pixels will preferably be different to the first group of pixels. The second group of pixels may comprise all the pixels from an area of interest. The second group of pixels may comprise a subgroup of the pixels from an area of interest, i.e. a lower number of pixels than the number of pixels in the area of interest. The second group of pixels may comprise more pixels than the pixels in an area of interest. The second group of pixels may comprise a larger number of pixels than the first group of pixels. The second group of pixels may comprise a lower number of pixels than the first group of pixels. The second group of pixels may comprise pixels which are part of the pixels from the first group of pixels. The second group of pixels may comprise pixels which are not part of the pixels from the first group of pixels.

The first and/or second group of pixels may be selected from a processed version of the one or more X-ray images, e.g. a filtered version. Additionally or alternatively, the first and/or second group of pixels may be selected from the original version of the one or more X-ray images, based on an area of interest found in the one or more X-ray images. Additionally or alternatively, the first and/or second group of pixels may be selected from a X-ray image from the one or more X-ray images, based on an area of interest found in another X-ray image from the one or more X-ray images.

The processing of the first and/or second group of pixels to determine the first and/or second parameter value may involve using a mathematical and/or statistical function to determine the first and/or second parameter values. Additionally or alternatively, processing of the first and/or second group of pixels may involve applying one or more filters to the first and/or second group of pixels. Such filters may be the same type of filter as used in the first processing step or another type of filter e.g. a Sobel filter or Laplacian filter. The one or more filters may be applied to an X-ray image before selecting the first and/or second group of pixels from the X-ray image, or alternatively be applied directly to each group of pixels.

Classifying of the one or more X-ray images may be done by comparing the first and/or second parameter value to a threshold value. The threshold value may be a maximum or a minimum value. Classifying may involve determining that the first and/or second parameter values are below or above the threshold value. Classifying may involve determining that the first and/or second parameter values are within a range defined by two threshold values, a minimum threshold value and maximum threshold value, respectively. Additionally or alternatively, classifying may involve determining that the first and/or second parameter value are outside a range defined by two threshold values. Additionally or alternatively, classifying may involve determining that the first and/or second parameter value are both outside one range defined by two threshold values and within another range defined by two other threshold values.

The threshold values for the first parameter value and the second parameter value (and potential further parameter values) for a particular food product may be found experimentally. As an example, a training data set may be used containing a number of X-ray images of a particular food product such as 50, 100 or 200 images. Increasing the number of images will increase how precise a threshold may be determined. The training data set should preferably comprise a suitable mixture of X-ray images of the food product comprising a particular unwanted structure and X-ray images of the food product that do not contain the unwanted structure. It should be determined for each X-ray image if the food product contains an unwanted structure and where in the food product the unwanted structure is present. This may be done by manually inspecting the X-ray images, using an alternative imaging modality, and / or by manually dissecting the food products. The X-ray images of the training data set should be processed to find areas of interest. For each area of interest, the first parameter value and the second parameter value should be determined. The threshold values may then be set so that all areas of interest containing an unwanted structure are correctly identified with a needed statistical certainty.

In some embodiments the method comprises that at least one of the first and second parameter value satisfy any one of the above-described classifying scenarios, in order to classify an X-ray images of the one or more X-ray images as comprising an unwanted structure. In an alternatively embodiment, the method comprises that both the first and second parameter value satisfy any one of the above-described classifying scenarios, in order to classify an X-ray images of the one or more X-ray images as comprising an unwanted structure.

Additionally or alternatively, classifying of the one or more X-ray images may be done by a machine learning data architecture trained to identify one or more unwanted structures in an X-ray image based on the first and/or second parameter values.

The machine learning data architecture may be trained using a training data set. As an example, a training data set may be used containing a number of X-ray images of a particular food product such as 50, 100 or 200 images. Increasing the number of images will increase how precise a threshold may be determined. The training data set should preferably comprise a suitable mixture of X-ray images of the food product comprising a particular unwanted structure and X-ray images of the food product that do not contain the unwanted structure. It should be determined for each X-ray image if the food product contains a particular unwanted structure and where in the food product the unwanted structure is present. This may be done by manually inspecting the X-ray images, using an alternative imaging modality, and / or by manually dissecting the food products. The X-ray images of the training data set should be processed to find areas of interest. For each area of interest, the first parameter value and the second parameter value should be determined. The machine learning data structure may then be trained by being provided with for each area of interest the first parameter value, the second parameter value and information about the presence of an unwanted structure in the particular area of interest.

In some embodiments, if an unwanted structure is determined by the method, the method further comprises indicating via an output device that an unwanted structure has been determined. The output device may give an audible or visual output, e.g. through a speaker, indicator light or screen.

In some embodiments, if an unwanted structure is determined by the method, the method may further comprises discarding the food product.

The method may be a computer implemented method.

In some embodiments, the method further comprising obtaining a first filtered version of the first group of pixels, and obtaining a first filtered version of the second group of pixels, and processing the first filtered version of the first group of pixels to determine a third parameter value, and processing the first filtered version of the second group of pixels to determine a fourth parameter value, wherein the classifying of the one or more X-ray images are further based on the third parameter value and fourth parameter value. By further basing the classifying step on the third parameter value and fourth parameter value from filtered versions of the first and second group pf pixels a higher probability of a true prediction of the presence of an unwanted structure may be achieved.

Obtaining and processing of the first filtered version of the first and second group of pixels may be done according to any previously described way. The step of obtaining the first filtered version the first and second group of pixels may involve applying a filter to the first and/or second group of pixels. A filter may be applied before the first and/or second group of pixels have been selected from the one or more X-ray images.

The filter used in order to create first filtered version of the first and second group of pixels may be a linear filter or non-linear filter. The filter used in order to create first filtered version of the first and second group of pixels may be a filter which detects edges or curvatures in an image, such as a Sobel filter, Laplacian filter, Canny filter, Prewitt filter, or Roberts filter. The filter used in order to create first filtered version of the first and second group of pixels may be the same filter as the filter used to filter the one or more X-rays images, i.e. a filter which passes high frequent spatial signals. The filter used in order to create first filtered version of the first and second group of pixels may be a filter which is not used on the one or more X-ray images in their entirety, but rather only used on a selected group of pixels. The filter used in order to create the first filtered version of the first and second group of pixels may be one type of filter for the first group of pixels and another type of filter for second group of pixels.

In some embodiments the second group of pixels surrounds or encompasses the first group of pixels. Thereby, allowing comparison of the surrounding in close proximity of the first group of pixels. Pixel information, i.e. degree of attenuation, may be compared between the first group of pixels and the second group of pixels. Differences in pixel information between the first group of pixels and the second group of pixels can reveal unwanted structures as the surroundings to an unwanted structure will give rise to a different pixel information than the unwanted structure itself.

For example, bone have a higher attenuation than soft tissue, such as muscle, adipose and connective tissue due to e.g. its higher Ca²⁺ content. Thus, pixel information for a group of pixels representing a bone is expected to have a high attenuation value and low variability within the group of pixels. When looking at a group of pixels that represent muscle, adipose and connective tissue, a lower attenuation value and higher variability is expected within the group of pixels.

Some structures in e.g. a food product, will typically have less of a difference in attenuation between the structure and its surroundings. As an example, patterned structures with a lot of tissue interfaces, such as a fish's stratified muscles tissue. In general, the muscle, connective and adipose tissue will have a similar degree of attenuation. However, the tissue interfaces in the patterned structures give rise to a higher attenuation value in the pixel information. Thus, when comparing two groups of pixels having a similar average attenuation and variability, normally occurring structures may be identified.

Thus, if an area of interest contains a bone, the first group of pixels may have a high average attenuation value and a low variability, and the second group of pixels may have a lower average attenuation value and a higher variability.

However, in some situation the structures in the food product is the unwanted structure. This is the case when trying to identify wood breast syndrome in poultry filets. Thus, if an area of interest contains wooden breast syndrome, both the first group of pixels and the second group of pixels may have a slightly elevated average attenuation value and a high variability. Thus, the method may be tuned to identify different unwanted structures.

The second group of pixels may surround the first group of pixels, either partially or completely. If the second group of pixels is chosen to surround the first group of pixels, the second group of pixels may abut, but not share, at least some of the pixels from the first group of pixels. Alternatively, second group of pixels may surround the first group of pixels without abutment of any of the pixels from the first group of pixels.

The second group of pixels may encompass the first group of pixels, either partially, or completely i.e. containing all of the pixels from the first group of pixels. Encompass may involve also partially or complete surrounding the first group of pixels, i.e. abutment and sharing of at least some of the pixels from the first group of pixels.

A group of pixels may be understood as a connected group of pixels with a common outer edge, where each pixel in the group of pixels abuts at least one nearest neighbor pixel from the eight neighboring pixels.

In some embodiments, the method further comprising selecting for each of the one or more areas of interest a third group of pixels and processing the third group of pixels to determine a fifth parameter value, wherein the classifying of the one or more X-ray images are further based on the fifth parameter value. By including a third group of pixels from which a fifth parameter value is determined, the accuracy of the classification is further improved.

In some embodiments, the method further comprising obtaining a first filtered version of the third group of pixels, and processing the first filtered version of the third group of pixels to determine a sixth parameter value, wherein the classifying of the one or more X-ray images are further based on the sixth parameter value. By further basing the classifying step on a filtered version of the third group of pixels and determining a sixth parameter value from the filtered version, a method with a higher predictability is provided.

In some embodiments the second group of pixels surrounds and do not encompass the first group of pixels and the third group of pixels encompasses both the first group of pixels and second group of pixels. Consequently, there is provided a way to compare pixel information from the first group of pixels, the pixels surrounding the first group of pixels and a combination thereof.

The first, second and third group of pixels, may represent the assumed unwanted structure, the surroundings of the assumed unwanted structure and a combination of the assumed unwanted structure and its surroundings.

A group of pixels may be understood as a connected group of pixels with a common outer edge, where each pixel in the group of pixels abuts at least one nearest neighbor pixel from the eight neighboring pixels.

The second group of pixels may either partially or completely surround the first group of pixels. The second group of pixels may abut at least some of the pixels from the first group of pixels. Alternatively, the second group of pixels may surround the first group of pixels without abutment of any of the pixels from the first group of pixels.

The third group of pixels may either partially or completely encompass the first and second group of pixels. The third group of pixels may partially encompass the first group of pixels, and completely encompass the second group of pixels, or vice versa. The third group of pixels may in its entirety be made up of pixels from the first and second group of pixels. Alternatively, the third group of pixels may comprise pixels which is not part of the first and/or second group of pixels.

Encompassing may involve also partially or complete surrounding the first and/or the second group of pixels, i.e. some of the pixels from the third group of pixels surrounds all of the pixels of the first and/or the second group of pixels, while the remaining pixels from the third group of pixels overlap with pixels of the first and/or the second group of pixels.

In some embodiments at least one of the determined parameter values relates to the variability of its respective group of pixels. Variability characteristic relating to known unwanted structures can be correlated with determined parameter values to aid in determining the presence of an unwanted structure.

Any measure of variability may be used in analysis of a respective group of pixels. Examples of parameters values which relates to variability may e.g. be variance, standard deviation, interquartile range or the range between pixels values.

The unwanted structures in a food product will in many cases have a high degree of attenuation and uniformity within the unwanted structure, thus a low variability is expected within a group of pixels that indeed is an unwanted structure, while a high variability is expected when comparing two groups of pixels being an unwanted structure and its surroundings, respectively.

In some embodiments at least one of the determined parameter values relates to the average intensity of its respective group of pixels. Average intensity characteristic relating to known unwanted structures can be correlated with determined parameter values to aid in determining the presence of an unwanted structure.

Any measure of average intensity may be used in analysis of a respective group of pixels. Examples of parameters values which relates to average intensity may e.g. be arithmetic mean, median value, midrange value or mode.

In some embodiments the step of processing the one or more X-ray images to find one or more areas of interest comprises applying a high-pass filter to the one or more X-ray images. Consequently, unwanted structures can be found in an X-ray image by looking for edges of the unwanted structures which typically will give rise to high frequent spatial signals.

In some embodiments the food product is at least one of fish and poultry. Thereby providing a method which replace manual handling and determination of unwanted structures in fish and/or poultry. The provided method may improve on production quality and throughput.

In some embodiments the unwanted structure is a fish bone.

In some embodiments the unwanted structure is caused by wooden breast syndrome in poultry.

In some embodiments the unwanted structure is a bone in poultry, such as a fan bone or pin bone. As poultry has a lot of pneumatic bones, detection of these may advantageously be based on edge detection in an X-ray image of a poultry meat sample which might comprise such an unwanted bone.

According to a second aspect, the invention relates to a computer program product comprising program code means adapted to cause a data processing system to perform the steps of the method according to first aspect, when said program code means are executed on the data processing system. The computer program product may provide identical or similar advantages to the method of the first aspect of this disclosure. Embodiments of the computer program product may be the same as described with respect to the method according to the first aspect of this disclosure.

The data processing system may be and/or may comprise a memory and a processing unit.

In some embodiments the computer program product comprises a non-transitory computer-readable medium having stored thereon the program code means.

According to a third aspect, the invention relates to an X-ray image processing apparatus for detecting an unwanted structure in one or more X-ray images of a food product, the X-ray image processing apparatus comprising a processing unit configured to perform the method according to first aspect. The X-ray image processing apparatus may provide identical or similar advantages to the method of the first aspect of this disclosure. Embodiments of the X-ray image processing apparatus may be the same as described with respect to the method according to the first aspect of this disclosure.

Here and in the following, the term 'processing unit' is intended to comprise any circuit and/or device suitably adapted to perform the functions described herein. In particular, the above term comprises general purpose or proprietary programmable microprocessors, Digital Signal Processors (DSP), Application Specific Integrated Circuits (ASIC), Programmable Logic Arrays (PLA), Field Programmable Gate Arrays (FPGA), special-purpose electronic circuits, etc., or a combination thereof.

According to a fourth aspect, the invention relates to a system for detecting an unwanted structure in one or more X-ray images of a food product, the system comprising; an X-ray apparatus configured to obtaining one or more X-ray images of the food product, and a processing unit operationally connected to the X-ray apparatus and configured to detecting an unwanted structure in the one or more obtained X-ray images by performing the method according to any one of the claims 1 - 12. The system may provide identical or similar advantages to the method of the first aspect of this disclosure. Embodiments of the system may be the same as described with respect to the method according to the first aspect of this disclosure.

In some embodiments the system further comprises an output device configured to give an indication when the presence of an unwanted structure has been determined in the step of classifying the one or more X-ray images. Thereby providing a way for the system to notify personnel that an unwanted structure has been determined in a food product.

The output device may give indication via an audible or visual output, e.g. through a speaker, indicator light or display.

The indication may prompt personnel to manually discard or further inspect the food product. Additionally or alternatively, an displayed visual output may include an estimated location of the unwanted structure in the X-ray image of the food product, thereby guiding the personnel in inspecting or possibly removing the unwanted structure from the food product.

In some embodiments the system further comprises means for rejecting food products in which an unwanted structure has been determined. Thereby providing a way for the system to automatically discard food product determined to contain an unwanted structure.

The means for rejecting of food products may be a downstream conveyor belt, robot, or diverter, configured for sorting of moving food products.

In some embodiments the system further comprises a conveyor belt for conveying the food product to or from the X-ray apparatus. Thereby providing a way for the system to automatically transport food product to and from X-ray apparatus.

A person skilled in the art will appreciate that any one or more of the different aspects of the present invention can be implemented in different ways including a method, and a system for detecting an unwanted structure in one or more X-ray images of a food product as described above and in the following, each yielding one or more of the benefits and advantages described in connection with at least one of the aspects described above, and each having one or more preferred embodiments corresponding to the preferred embodiments described in connection with at least one of the aspects described above and/or disclosed in the dependent claims. Furthermore, it will be appreciated that embodiments described in connection with one of the aspects described herein may equally be applied to the other aspects.

### Brief description of the drawings

The above and/or additional objects, features and advantages of the present invention, will be further elucidated by the following illustrative and non-limiting detailed description of embodiments of the present invention, with reference to the appended drawings, wherein:
Fig. 1 shows an exemplary flow chart of an embodiment of a method according to the invention,
Fig. 2a-2c shows exemplary X-ray images of a food product.
Fig. 3a-3c shows an exemplary group of pixels from an area of interest.
Fig. 4 shows a schematic block diagram of an embodiment of a system according to the invention.

Similar reference numerals are used for similar elements across the various embodiments and figures described herein.

### Detailed description

In the following description, reference is made to the accompanying figures, which show by way of illustration how the invention may be practiced.

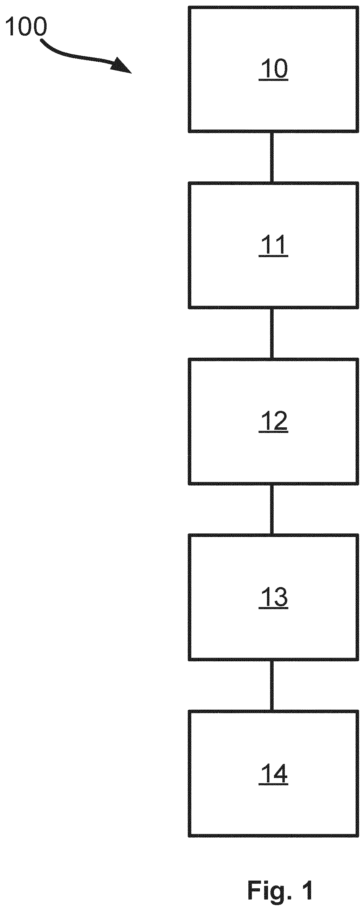

Fig. 1 shows an exemplary flow chart of an embodiment of a method 100 for detecting an unwanted structure in one or more X-ray images 31 of a food product 30 according to the invention.

The method 100 comprising obtaining 10 one or more X-ray images 31;
processing 11 the one or more X-ray images 31 to find one or more areas of interest 40, each potentially comprising an unwanted structure 41;
selecting 12 for each of the one or more areas of interest 40 a first group of pixels 50 and a second group of pixels 51;
processing 13 the first group of pixels 50 to determine a first parameter value and processing the second group of pixels 51 to determine a second parameter value; and
classifying 14 the one or more X-ray images based on the first parameter value and the second parameter value, to determine the presence of an unwanted structure 41.

The method 100 may be implemented in or by a processing unit 21, such as a DSP or the like.

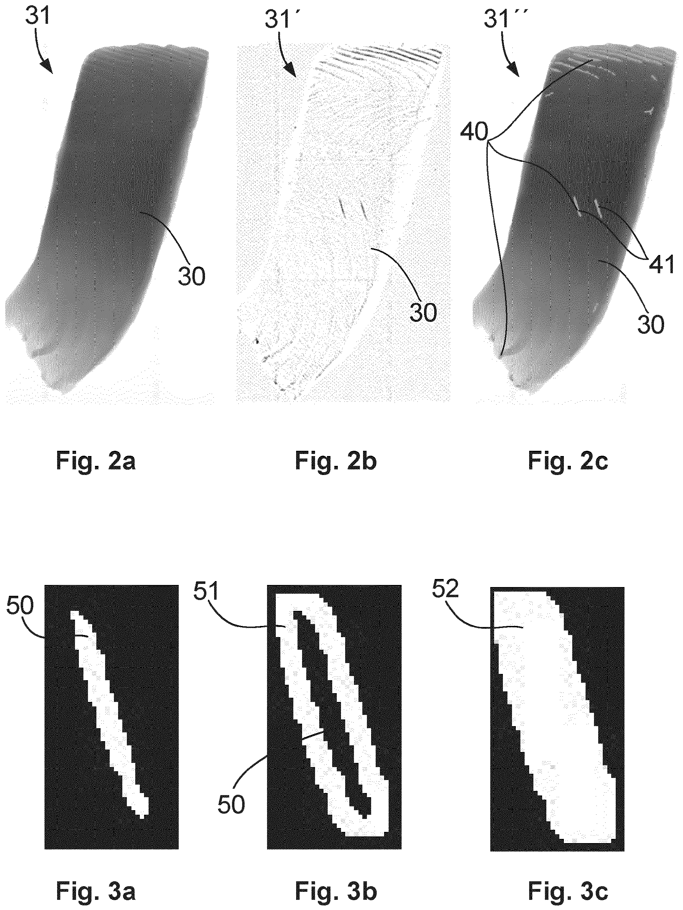

Fig. 2a-2c shows an exemplary X-ray image 31 of a food product 30. In this example, the food product 30 is salmon fillet, however a person skilled in the art will appreciate that other food products, such as other fish product or chicken breast fillets might as well be used. Referring first to fig. 2a which shows an obtained X-ray image 31 of the salmon fillet. Referring further to fig. 2b, which shows a processed version of the X-ray image 31' from fig. 2a. The processing in this example involved applying a high-pass filter to the original X-ray image 31 which yield the processed X-ray image 31' containing high frequent information of the original X-ray image 31 from fig. 2a. A threshold is applied to the processed X-ray image 31' to obtain a binary image in which each pixels with values over the threshold value remain and each connected group of pixels represents an area of interest 40, i.e. a potential unwanted structure 41. Information of position of the area of interests from the binary image is used to indicate in the original X-ray image, the areas of interest 40 marked with green color in fig. 2c. The indicated areas of interest 40 are used to select groups of pixels from the original X-ray image 31 for further processing.

Fig. 3a shows a first group of pixels 50 representing one of the areas of interest 40 selected in the previous step.

Fig. 3b shows a second group of pixels 51 surrounding the first group of pixels 50. In this example the second group of pixels 51 is selected by including pixels arranged withing a particular distance to the first group of pixels. As an example, the particular distance may dependent on the first group of pixels e.g. be dependent on the width of the first group of pixels. Alternatively, the distance may be preselected.

The first group of pixels 50 is processed to determine a first parameter value and the second group of pixels 51 is processed to determine a second parameter value.

The first and second parameter value may then compare to reveal differences and similarities of the first group of pixels 50 the second group of pixels 51. The first and second parameter value may for example be a calculation of the arithmetic mean or the median of the pixel groups, or alternatively a calculation of a standard deviation or variance, relating to the variability within the groups of pixels.

The first and second parameter value are used to determine if the area of interest 40 is indeed an unwanted structure or not. If an area of interest in the X-ray image is determined to be an unwanted structure the X-ray image will be classified as containing the presence of an unwanted structure.

In some embodiments a filter is applied, either to the X-ray image under investigation or to the selected first and second group of pixels 50, 51. Thus, creating a filtered version of the first and second group of pixels, which are used to determine a third and fourth parameter value. The third and fourth parameter value may be the same type of parameter value or a different type of parameter value as the first and second parameter value. The third and fourth parameter value are used to further increase the certainty in determining if the area of interest 40 is an unwanted structure or not.

In some embodiments a third group of pixels 52 is selected, as exemplified in fig. 3c. In the present example the third group of pixels 52 is a combination of the first and second group of pixels. The third group of pixels 52 is processed to determine a fifth parameter value. The fifth parameter value may be the same type of parameter value or a different type of parameter value as any of the previously discussed parameter values. The fifth parameter value is used to further increase the certainty in determining if the area of interest 40 is an unwanted structure or not.

A filtered version of the third group of pixels may be obtained in a similar fashion as described above. The filtered version of the third group of pixels is used to determine a sixth parameter value to further strengthen the determination of the presence of an unwanted structure.

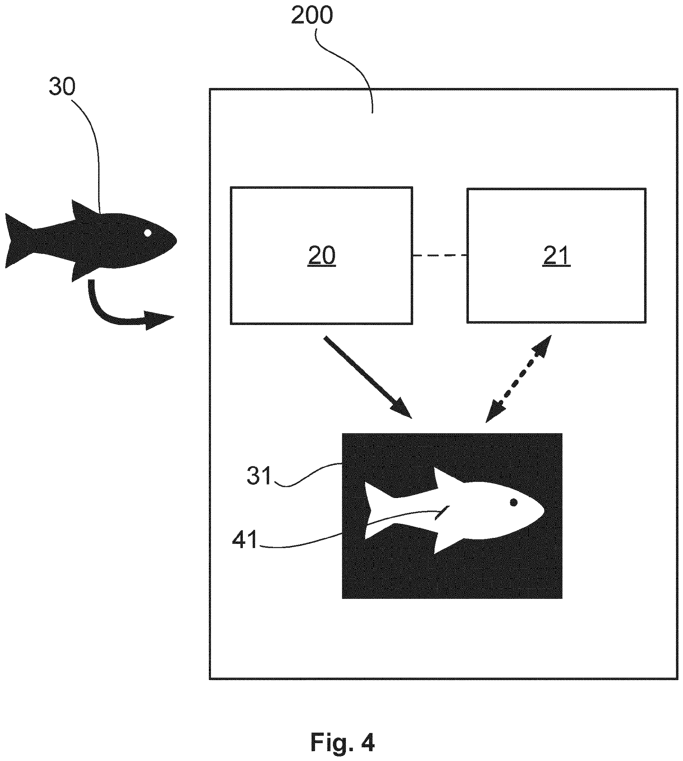

Lastly, referring to fig. 4, which shows a system 200 for detecting an unwanted structure 41 in one or more X-ray images 31 of a food product 30, the system comprising; an X-ray apparatus 20 configured for obtaining one or more X-ray images of the food product, and a processing unit 21 operationally connected to the X-ray apparatus and configured for detecting an unwanted structure in the one or more obtained X-ray images by performing the method 100 described with reference to fig. 1-3.

The illustrated food product 30 in fig. 4 is depicted as a whole fish, this is only meant to be illustrative and a person skilled in the art will understand that any food product may be used with the system and method according to the invention.

The processing unit 21 is configured to perform the method 100. Specifically, the processing unit 21 receives the one or more X-ray images 31 and outputs the determination of the presence of an unwanted structure for each food product which is provided to the system.

A food product may for example be provided to the system via a conveyor belt up stream of the system 200, which transports the food product to the system 200. If the processing unit 21 of the system 200 has made the determination that an unwanted structure is present in the food product, the food product 30 may be discarded or transported to a work station for further examination by use of a sorting conveyor belt downstream of the system 200.

One conceivable use case for the method and/or system is to find small bones in salmon fillet, which are left in the salmon fillet after processing of the whole fish into fillets. In such a case the fish bone will have a higher attenuation than muscle, adipose and connective tissue in the salmon. Even so, it is no trivial task to find small fish bones in salmon using X-ray, as muscles in salmon are arranged in lamellae structures, each separated by regions of connective and adipose tissue. The connective and adipose tissue between the muscles resembles the small bone structures in X-rays images. By comparing parameter values based on attenuation information of two or more pixels groups extracted from areas of interest the invention provides a way to distinguish between unwanted bones and normally occurring lamellae tissue in the salmon.

Another conceivable use case for the method and/or system is to determine if a chicken breast fillet comprises scar tissue as a consequence of wooden breast syndrome. In such a case the scar tissue, which is essentially a high concentration of connective tissue will have a similar attenuation to the surrounding muscle tissue. However, no normally occurring tissue in the chicken will have the same structural arrangement, thereby providing a way to identify areas of interest. Comparison of parameter values based on attenuation information of two or more pixels groups extracted from areas of interest provides a way to confirm whether the area of interest is due to wooden breast syndrome.

An alternative, or additional use case when examining chicken meat samples, is determination of bones in the chicken meat. Typical bones that are hard to find using conventional methods are the fan bones and pin bones. By using the disclosed method and/or system to determine if a chicken meat sample comprises unwanted bones, such as a fan bone or pin bone, a higher detection rate may be achieved. The bones may have a slightly higher attenuation than muscle, adipose and connective tissue in the chicken and further a lower variability. Thus, by comparing parameter values based on attenuation information of two or more pixels groups extracted from an area of interest, e.g. a first pixel group originating from a potential bone and a second pixel group originating from an area immediately surrounding the potential bone, the invention provides a way to distinguish between unwanted bones and normally occurring tissue in the chicken.

Although some embodiments have been described and shown in detail, the invention is not restricted to them, but may also be embodied in other ways within the scope of the subject matter defined in the following claims. In particular, it is to be understood that other embodiments may be utilized, and structural and functional modifications may be made without departing from the scope of the present invention.

It is emphasized that the term "comprises/comprising" when used in this specification is taken to specify the presence of stated features, steps or components but does not preclude the presence or addition of one or more other features, integers, steps, components or groups thereof.

## Claims

1. A method for detecting an unwanted structure in one or more X-ray images of a food product, the method comprising:
obtaining one or more X-ray images;
processing the one or more X-ray images to find one or more areas of interest, each potentially comprising an unwanted structure;
selecting for each of the one or more areas of interest a first group of pixels and a second group of pixels;
processing the first group of pixels to determine a first parameter value and processing the second group of pixels to determine a second parameter value; and
classifying the one or more X-ray images based on the first parameter value and the second parameter value, to determine the presence of an unwanted structure.

2. The method according to claim 1, further comprising obtaining a first filtered version of the first group of pixels, and obtaining a first filtered version of the second group of pixels, and processing the first filtered version of the first group of pixels to determine a third parameter value, and processing the first filtered version of the second group of pixels to determine a fourth parameter value, wherein the classifying of the one or more X-ray images are further based on the third parameter value and fourth parameter value.

3. The method according to claim 1 or 2, wherein the second group of pixels surrounds or encompasses the first group of pixels.

4. The method according to any one of the preceding claims, further comprising selecting for each of the one or more areas of interest a third group of pixels and processing the third group of pixels to determine a fifth parameter value, wherein the classifying of the one or more X-ray images are further based on the fifth parameter value.

5. The method according to claim 4, further comprising obtaining a first filtered version of the third group of pixels, and processing the first filtered version of the third group of pixels to determine a sixth parameter value, wherein the classifying of the one or more X-ray images are further based on the sixth parameter value.

6. The method according to claim 4 or 5, wherein the second group of pixels surrounds and do not encompass the first group of pixels and wherein the third group of pixels encompasses both the first group of pixels and second group of pixels.

7. The method according to claim 5 or 6, wherein at least one of the determined parameter values relates to the variability of its respective group of pixels.

8. The method according to any one of claim 5 to 7, wherein at least one of the determined parameter values relates to the average intensity of its respective group of pixels.

9. The method according to any one of the preceding claims, wherein the step of processing the one or more X-ray images to find one or more areas of interest comprises applying a high-pass filter to the one or more X-ray images.

10. The method according to any one of the preceding claims, wherein the food product is fish and the unwanted structure is fish bone; or the food product is poultry, and the unwanted structure is at least one of a pin bone, a fan bone and wooden breast syndrome.

11. An X-ray image processing apparatus for detecting an unwanted structure in one or more X-ray images of a food product, the X-ray image processing apparatus comprising a processing unit configured to perform the method according to any one of the claims 1 - 10.

12. A system for detecting an unwanted structure in one or more X-ray images of a food product, the system comprising:
an X-ray apparatus configured to obtaining one or more X-ray images of the food product, and
a processing unit operationally connected to the X-ray apparatus and configured to detecting an unwanted structure in the one or more obtained X-ray images by performing the method according to any one of the claims 1 - 10.

13. The system according to claim 12, further comprising a output device configured to give an indication when the presence of an unwanted structure has been determined in the step of classifying the one or more X-ray images.

14. The system according to claim 12 or 13, further comprising means for rejecting food products in which an unwanted structure has been determined.

15. A computer program product comprising program code means adapted to cause a data processing system to perform the steps of the method according to any one of claims 1 to 10, when said program code means are executed on the data processing system.
